# EUROPEAN PATENT APPLICATION

(11) **EP 1 736 155 A1**
(43) Date of publication of application: **27.12.2006**
(21) Application number: 05730546.8
(22) Date of filing: 13.04.2005
(51) Int. Cl.: A61K 31/4375, A61K 31/27, A61K 31/445, A61K 31/473, A61K 31/55, A61K 45/00, A61P 9/10, A61P 25/00, A61P 25/02, A61P 25/14, A61P 25/16, A61P 25/28, A61P 43/00, C07D 471/04

(54) **MEDICINE COMPRISING COMBINATION OF ACETYLCHOLINE ESTERASE INHIBITOR AND 5-SUBSTITUTED 3-OXADIAZOLYL-1,6-NAPHTHYRIDIN-2(1H)-ONE DERIVATIVE**

(30) Priority: 15.04.2004 JP 2004120406
(71) Applicant: Dainippon Sumitomo Pharma Co., Ltd., Osaka 541-8524 (JP)
(72) Inventor: FURUKAWA, Kiyoshi, Shiga 5200531 (JP); KURUMIYA, Satoshi c/o Dainippon Sumitomo Pharma,, Chuo-ku, Tokyo 104-8356 (JP); HASHIMOTO, Takashi c/o Dainippon Sumitomo Pharma,, Suita-shi, Osaka 564-0053 (JP)
(74) Representative: Coleiro, Raymond
(86) International application number: PCT/JP2005/007159
(87) International publication number: WO 2005/099696

(57) **Abstract**

The invention provides a medicine comprising a combination of an acetylcholinesterase inhibitor and a 5-substituted-3-oxadiazolyl-1,6-naphthyridin-2(1H)-one derivative of the formula (I): 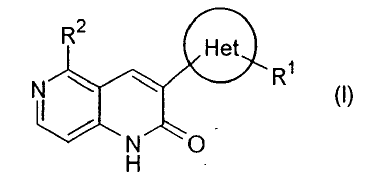 wherein Het is oxadiazolyl; R¹ is hydrogen atom, lower alkyl, lower cycloalkyl, lower alkenyl, lower alkoxy, substituted or unsubstituted aryl, substituted or unsubstituted heteroaryl, etc., and R² is hydrogen atom, lower alkyl, a lower cycloalkyl, substituted or unsubstituted aryl, etc., which is useful for treating neuropsychiatric disease-accompanying dysmnesia and other cognitive impairment; a method for treating the above-mentioned diseases; and use thereof.

## Description

### TECHNICAL FIELD

The invention relates to a medicine comprising a combination of an acetylcholinesterase inhibitor and a 5-substituted-3-oxadiazolyl-1,6-naphthyridin-2(1H)-one derivative or a physiologically acceptable acid addition salt thereof; a method for treating dysmnesia (memory disorder) and other cognitive impairment associated with neuropsychiatric diseases comprising administering the medicine; and use thereof.

### BACKGROUND ART

With the advance of the aging of society, the increase of patients suffering from senile dementia has been causing a social problem. About 90 % of patients with senile dementia is occupied with Alzheimer-type dementia and vascular dementia caused by cerebrovascular disorder. With respect to the symptoms of dementia, short- or long-term dysmnesia is observed as a fundamental and common core symptom. In the patients suffering from dementia, the functions of various neurotransmitter systems are remarkably decreased mainly in the cerebral cortex and the limbic system, and the function of cerebral energy metabolism is also decreased. Especially Alzheimer's disease is a progressive neurodegenerative disorder whose symptom is mainly attenuation and decline of memory, and the functions of two or more neurotransmitter systems in the patients with Alzheimer's disease such as cholinergic, glutamatergic, γ-aminobutyric acid (hereinafter, abbreviated to "GABA"), neuropeptidergic and monoaminergic systems are decreased, therefore, it is presumed that the main cause of cognitive impairment is dysfunction of such neurotransmitter systems [see Coyle, J. T., et al., "Science," (US) 1983, Vol. 219, p.1184-1190; and Gottfries, C. G., et al., "Psychopharmacology," (DE) 1985, Vol. 86, p.245-252].

Based on the above-mentioned presumption, especially on the knowledge that the marked dysfunction of cholinergic neurotransmitter system is a cause of cognitive impairment, some medicaments that ameliorate symptoms of dementia by activating the cholinergic system have been developed, and now donepezil hydrochloride (hereinafter, abbreviated to just "donepezil"), tacrine hydrochloride (hereinafter, abbreviated to just "tacrine"), rivastigmine tartrate (hereinafter, abbreviated to just "rivastigmine"), galantamine hydrobromide (hereinafter, abbreviated to just "galantamine") and so on are used in clinical applications. These medicaments inhibit acetylcholinesterase, an enzyme that catabolizes acetylcholine, and thereby can activate intracerebral cholinergic neurons. Donepezil, one of the representative medicaments among them, is known to ease cognitive impairment relating to Alzheimer's disease (e.g., see "PDR Generics," (US) MEDICAL ECONOMICS COMPANY 1998, p.960-964), and lately it is also reported that the medicament is efficient for cognitive impairment caused by vascular dementia (e.g., see Wilkinson, D., et al., "Neurology," (US) 2003, Vol. 61, No. 4, p.479-486).

However, it is known that an acetylcholinesterase inhibitor has a side effect such as convulsions, nausea/vomiting, diarrhea, hypersalivation, sweating, anxiety and insomnia, and the dissociation between the efficacy and the side-effect thereof is not so sufficient.

Actually, Palmer, A. M., "Trends in Pharmacological Science," (NL) 2002, Vol. 23, No. 9, p. 426-433 discloses efficacies and side-effects of various acetylcholinesterase inhibitors; for example, tacrine exhibits effects of ameliorating ADAS-cog. (Alzheimer's disease assessment scale-cognitive subscale) and MMSE (mini mental state examination) but it also exhibits hepatotoxicity and gastrointestinal dysfunction as side effects. In addition, the reference discloses that donepezil exhibits effects of ameliorating ADAS-cog., MMSE, CIBIC (clinician's interview-based impression of change scale) and Global clinical state, but it also exhibits nausea, vomiting, or diarrhea as side effects; rivastigmine exhibits effects of ameliorating ADAS-cog. and global clinical state, but it also exhibits nausea, diarrhea and hypophagia as side effects; and galantamine exhibits effects of ameliorating ADAS-cog., global impression and activities of daily living, but it also exhibits nausea, diarrhea and acute hypophagia as side effects.

Besides, there are also some attempts to develop benzodiazepine (hereinafter, optionally abbreviated to "BZP") receptor inverse agonists as the therapeutic agent for ameliorating symptoms of dementia. Heretofore, many studies have been done on the relationship between the binding-manner to BZP receptors and the pharmacological activity thereof, and then BZP receptor agonists have been developed as antianxiety drugs, antidepressant drugs, drugs for sleep disorder, antiepileptic drugs and so on, since the BZP receptor agonist might modify functions of the GABA-A receptor. However, it is known that the BZP receptor agonist causes dysmnesia (amnesia) as a side effect. On the other hand, the BZP inverse agonist is expected to have the anti-dysmnesia action (anti-amnesia action) and to activate the cerebral function, since it is known that the inverse agonist exhibits opposite actions to those of the BZP receptor agonist and enhances the cholinergic activity which is considerably related with the cognitive function.

As an example of such compounds, WO 99/003857 discloses 5-substituted-3-oxadiazolyl-1,6-naphthyridin-2(1H)-one derivatives of the following formula (I): 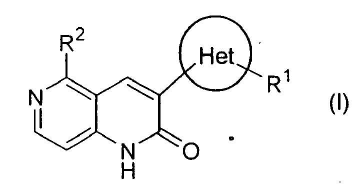 wherein Het is an oxadiazolyl group;
R¹ is hydrogen atom, a lower alkyl group, a lower cycloalkyl group, trifluoromethyl group, a lower alkenyl group, a lower alkynyl group, a lower alkoxy group, a lower alkoxy-lower alkyl group, a hydroxy-lower alkyl group, a substituted or unsubstituted aryl group, or a substituted or unsubstituted heteroaryl group; and
R² is hydrogen atom, a lower alkyl group, a lower cycloalkyl group, a lower cycloalkylmethyl group, a lower alkenyl group, a lower cycloalkenyl group, a lower alkynyl group, a substituted or unsubstituted aryl group, or a substituted or unsubstituted heteroaryl group, which also discloses that said compounds exhibit selective and high affinity for the BZP receptor and especially also have properties as the inverse agonist and hence they are expected to be useful as the medicament for treating dysmnesia associated with senile dementia, vascular dementia and Alzheimer-type dementia or as the cerebral function enhancer.

In addition, for the purpose of ameliorating symptoms of dementia, the development of medicines for preventing or ameliorating hypofunction of the N-methyl-D-aspartic acid (hereinafter, abbreviated to "NMDA") receptor have been also tried focusing on the decrease of glutamatergic neuron and NMDA receptor which is one of receptors of glutamic acid.

WO 01/98300 discloses 5-substituted-3-oxadiazolyl-1,6-naphthyridin-2(1H)-one derivatives of the formula (I) as a example of the above mentioned medicines. It also discloses that the compound exhibits a remarkably potent effect of delaying or preventing the progress of neuronal degeneration caused by hypofunction of the NMDA receptor and hence in mammals (including human being) they are useful for preventing and/or treating neurodegenerative and neuropsychiatric disorders associated with hypofunction of the NMDA receptor, such as Alzheimer's disease and schizophrenia (schizophrenic disorder), respectively.

### DISCLOSURE OF INVENTION

### Problem to be solved by the invention

It has been desired to research and develop a medicine that has a potent effect for treating dysmnesia (memory disorder) and cognitive impairment associated with neurodegenerative disorder such as Alzheimer's disease and cerebrovascular disorder, and also has an excellent property such as markedly low side-effects.

### Means to solve the problem

The present inventors have found that use of a combination comprising an acetylcholinesterase inhibitor and a 5-substituted-3-oxadiazolyl-1,6-naphthyridin-2(1H)-one derivative of the following formula (I) which is a benzodiazepine receptor inverse agonist can exhibit an unexpectedly potent therapeutic effect (promnesic effect) and the present invention has been completed based upon the new finding. That is, the invention provides a medicine comprising a combination of an acetylcholinesterase inhibitor and at least one compound selected from 5-substituted-3-oxadiazolyl-1,6-naphthyridin-2(1H)-one derivatives of the formula (I): 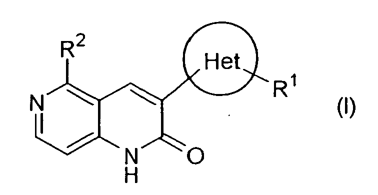 wherein Het is an oxadiazolyl group;
R¹ is hydrogen atom, a lower alkyl group, a lower cycloalkyl group, trifluoromethyl group, a lower alkenyl group, a lower alkynyl group, a lower alkoxy group, a lower alkoxy-lower alkyl group, a hydroxy-lower alkyl group, a substituted or unsubstituted aryl group, or a substituted or unsubstituted heteroaryl group; and
R² is hydrogen atom, a lower alkyl group, a lower cycloalkyl group, a lower cycloalkylmethyl group, a lower alkenyl group, a lower cycloalkenyl group, a lower alkynyl group, a substituted or unsubstituted aryl group, or a substituted or unsubstituted heteroaryl group, and physiologically acceptable acid addition salts thereof.

The 5-substituted-3-oxadiazolyl-1,6-naphthyridin-2(1H)-one derivative of the formula (I) used herein includes as a preferable example a compound of the formula (I) wherein R¹ is a C₁-C₃ alkyl group, a C₃-C₄ cycloalkyl group, or a C₂-C₃ alkenyl group; R² is hydrogen atom, a C₁-C₄ alkyl group, a C₃-C₆ cycloalkyl group, a substituted or unsubstituted aryl group, or a substituted or unsubstituted heteroaryl group. More preferable example of the compound includes the compound of the formula (I) wherein R¹ is a C₁-C₃ alkyl group or a C₃-C₄ cycloalkyl group; R² is hydrogen atom, a C₁-C₃ alkyl group, a C₃-C₄ cycloalkyl group, a substituted or unsubstituted phenyl group, or a substituted or unsubstituted heteroaryl group. Even more preferable examples of the compound include the following compounds.
3-(5-Ethyl-1,2,4-oxadiazol-3-yl)-5-(2-methylcyclopropyl)-1,6-naphthyridin-2(1H)-one,
3-(5-methyl-1,2,4-oxadiazol-3-yl)-5-(2-methylphenyl)-1,6-naphthyridin-2(1H)-one,
3-(5-methyl-1,2,4-oxadiazol-3-yl)-5-(3-methoxyphenyl)-2,6-naphthyridin-2(1H)-one,
3-(5-methyl-1,2,4-oxadiazol-3-yl)-5-(4-methoxyphenyl)-1,6-naphthyridin-2(1H)-one,
3-(5-ethyl-1,2,4-oxadiazol-3-yl)-5-(2-thienyl)-1,6-naphthyridin-2(1H)-one,
3-(5-methyl-1,2,4-oxadiazol-3-yl)-5-(4-pyridyl)-1,6-naphthyridin-2(1H)-one,
3-(3-ethyl-1,2,4-oxadiazol-5-yl)-5-methyl-1,6-naphthyridin-2(1H)-one,
3-(3-ethyl-1,2,4-oxadiazol-5-yl)-5-(3-fluorophenyl)-1, 6-naphthyridin-2(1H)-one,
3-(3-methyl-1,2,4-oxadiazol-5-yl)-5-(3-methylphenyl)-1,6-naphthyridin-2(1H)-one,
3-(3-methyl-1,2,4-oxadiazol-5-yl)-5-(3-methoxyphenyl)-1,6-naphthyridin-2(1H)-one,
3-(3-ethyl-1,2,4-oxadiazol-5-yl)-5-(9-methoxyphenyl)-1,6-naphthyridin-2(1H)-one,
3-(3-ethyl-1,2,4-oxadiazol-5-yl)-5-(4-pyridyl)-1,6-naphthyridin-2(1H)-one, and
3-(3-cyclopropyl-1,2,4-oxadiazol-5-yl)-5-(3-thienyl)-1,6-naphthyridin-2(1H)-one.

In addition, the present invention provides a method for treating dysmnesia and cognitive impairment associated with neuropsychiatric diseases, which comprises administering a therapeutically effective amount of a medicine comprising a combination of an acetylcholinesterase inhibitor and at least one compound selected from 5-substituted-3-oxadiazolyl-1,6-naphthyridin-2(1H)-one derivatives of the formula (I) and physiologically acceptable acid addition salts thereof to a patient suffering therefrom.

In addition, the present invention provides a method for treating Alzheimer's disease, which comprises administering a therapeutically effective amount of a medicine comprising a combination of an acetylcholinesterase inhibitor and at least one compound selected from 5-substituted-3-oxadiazolyl-1,6-naphthyridin-2(1H)-one derivatives of the formula (I) and physiologically acceptable acid addition salts thereof to a patient in need of such treatment.

The physiologically acceptable acid addition salt of the compound of the formula (I) includes, for example, inorganic acid salts such as hydrochloride, hydrobromide, hydroiodide, sulfate, phosphate, etc. and organic acid salts such as oxalate, maleate, fumarate, malonate, lactate, malate, citrate, tartrate, benzoate, methanesulfonate, tosylate, etc.

The "lower alkyl group" and "lower alkyl" moiety used herein denote a straight chain or branched chain alkyl group having 1 to 6 carbon atoms and include, for example methyl group, ethyl group, propyl group, isopropyl group, butyl group, isobutyl group, tert-butyl group, pentyl group, and hexyl group.

The "lower cycloalkyl group" denotes a cycloalkyl group having 3 to 6 carbon atoms and includes, for example, cyclopropyl group, cyclobutyl group, cyclopentyl group, and cyclohexyl group, which may be optionally substituted by C₁ - C₃ alkyl group(s) or a halogen atom(s).

The "lower alkenyl group" and "lower alkynyl group" have a straight or branched carbon chain comprising 2 - 6 carbon atoms and include, for example, allyl group, 1-propenyl group, propargyl group, and 2-methyl-1-ethynyl group.

The "lower cycloalkenyl group" denotes a cycloalkenyl group having 5 to 6 carbon atoms and includes, for example, cyclohexenyl group.

The "lower alkoxy group" and "lower alkoxy" moiety denote a straight chain or branched chain alkoxy group having 1 to 6 carbon atoms and include, for example, methoxy group, ethoxy group, propoxy group, isopropyloxy group, butyloxy group, isobutyloxy group, tert-butyloxy group, pentyloxy group, and hexyloxy group.

The "aryl group" and "aryl" moiety denote a phenyl group or a naphthyl group, which may optionally have 1 - 3 substituents selected from halogen atoms, C₁ - C₃ alkyl groups, trifluoromethyl groups, hydroxy groups, C₁ - C₃ alkoxy groups, trifluoromethoxy groups, cyano groups, amino groups, and nitro groups.

The "heteroaryl group" denotes a 5- to 6-membered aromatic heterocyclic group containing 1 to 2 hetero atoms which are the same or different and are selected from nitrogen atoms, oxygen atoms and sulfur atoms, and includes, for example, furyl, thienyl, pyrrolyl, oxazolyl, isoxazolyl, pyridyl, pyridazinyl, and pyrimidinyl, wherein such heterocyclic group may optionally have 1 to 3 substituents selected from halogen atoms, C₁ - C₃ alkyl groups, hydroxy groups, C₁ - C₃ alkoxy groups and amino groups.

Further, the "halogen atom" denotes fluorine atom, chlorine atom, bromine atom or iodine atom.

The 5-substituted-3-oxadiazolyl-1,6-naphthyridin-2(1H)-one derivative of the formula (I) or the physiologically acceptable acid addition salt thereof, and the medicament comprising it (or them) as an active ingredient are described in WO 99/003857 or WO 01/98300 and can be prepared by the methods disclosed therein.

It is thought that the acetylcholinesterase inhibitor activates the intracerebral cholinergic neuron via inhibiting acetylcholinesterase that is a catabolic enzyme of acetylcholine and relieves cognitive impairment associated with Alzheimer's disease. The acetylcholinesterase inhibitor which can be used in the invention includes any acetylcholinesterase inhibitor known by a skilled person, and preferably commercially available ones, such as donepezil, tacrine, rivastigmine and galantamine, and preferably donepezil.

### EFFECT OF THE INVENTION

The concomitant (combined) administration comprising an acetylcholinesterase inhibitor and a 5-substituted-3-oxadiazolyl-1,6-naphthyridin-2(1H)-one derivative of the formula (I) or a physiologically acceptable acid addition salt thereof can produce more ameliorating effect than that expected by a single administration of each medicament. That is, the combination comprising both medicaments exhibits a potent synergistic ameliorating effect on dysmnesia caused by hypofunction of the cholinergic neuron.

In addition, a medicament comprising as an active ingredient a 5-substituted-3-oxadiazolyl-1,6-naphthyridin-2(1H)-one derivative of the formula (I) or a physiologically acceptable acid addition salt thereof also exhibits a markedly potent ameliorating effect on dysmnesia caused by hypofunction of the glutamatergic neuron which is not ameliorated by the acetylcholinesterase inhibitor. Therefore, the combination comprising an acetylcholinesterase inhibitor and a 5-substituted-3-oxadiazolyl-1,6-naphthyridin-2(1H)-one derivative of the formula (I) or a physiologically acceptable acid addition salt thereof can bring a synergetic or complementary effect on dysmnesia caused by hypofunction of the cholinergic or the glutamatergic neuron, and is quite useful.

In addition, the combination comprising each therapeutically effective amount of the both medicaments enhances the therapeutic efficacy of the acetylcholinesterase inhibitor synergically or complementarily, and consequently the amount of the inhibitor can be decreased. Thereby, side-effects of the acetylcholinesterase inhibitor can be reduced and additionally the diminution of the therapeutic effect of each medicament during long term administration can be suppressed.

### BEST MODE FOR CARRYING OUT THE INVENTION

According to the present invention, a medicine comprising a combination of an acetylcholinesterase inhibitor and a 5-substituted-3-oxadiazolyl-1,6-naphthyridin-2(1H)-one derivative of the formula (I) or a physiologically acceptable acid addition salt thereof can be administered to a patient for treating dysmnesia (memory disorder) and other cognitive impairments associated with neuropsychiatric diseases, especially dementia such as Alzheimer's disease and cerebrovascular disorder. The medicine comprising the combination of the invention is not especially limited as long as a medicine comprising an acetylcholinesterase inhibitor and a medicine comprising as an active ingredient a 5-substituted-3-oxadiazolyl-1,6-naphthyridin-2(1H)-one derivative of the formula (I) or a physiologically acceptable acid addition salt thereof are combined when they are administered.

The examples of such combined administration systems include 1) administration of a formulation comprising an acetylcholinesterase inhibitor and 5-substituted-3-oxadiazolyl-1,6-naphthyridin-2(1H)-one derivative(s) of the formula (I) or physiologically acceptable acid addition salt(s) thereof; 2) simultaneous administration of two formulations comprising separately an acetylcholinesterase inhibitor and 5-substituted-3-oxadiazolyl-1,6-naphthyridin-2(1H)-one derivative(s) of the formula (I) or physiologically acceptable acid addition salt(s) thereof via the same route; 3) time-lagged administration of two formulations comprising separately an acetylcholinesterase inhibitor and 5-substituted-3-oxadiazolyl-1,6-naphthyridin-2(1H)-one derivative(s) of the formula (I) or physiologically acceptable acid addition salt(s) thereof via the same route wherein the administration order of the both formulations is indefinite; 4) simultaneous administration of two formulations comprising separately an acetylcholinesterase inhibitor and 5-substituted-3-oxadiazolyl-1,6-naphthyridin-2(1H)-one derivative(s) of the formula (I) or physiologically acceptable acid addition salt(s) thereof via the different route; 5) time-lagged administration of two formulations comprising separately an acetylcholinesterase inhibitor and 5-substituted-3-oxadiazolyl-1,6-naphthyridin-2(1H)-one derivative(s) of the formula (I) or physiologically acceptable acid addition salt(s) thereof via the different route wherein the administration order of the both formulations is indefinite; etc. The above mentioned 2) or 3) is preferable among the above 1)-5).

The "route" used herein means oral, intravenous, intramuscular or percutaneous administration or other. In more detail, it is preferable that an acetylcholinesterase inhibitor and a 5-substituted-3-oxadiazolyl-1,6-naphthyridin-2(1H)-one derivative of the formula (I) or a physiologically acceptable acid addition salt thereof are formulated into different oral formulations such as tablet and the formulations are administered simultaneously or with a time lag.

Each medicament (formulation) used herein has low toxicity and thereby can be safely administered to a patient in an oral or parenteral manner. The dosage of each medicament of the invention in the combination of an acetylcholinesterase inhibitor and a 5-substituted-3-oxadiazolyl-1,6-naphthyridin-2(1H)-one derivative of the formula (I) or a physiologically acceptable acid addition salt thereof should be defined from the standard of general clinical use and can be selected optionally considering the subject to be administered, age and weight of the subject, symptom thereof, administration time , type of formulation, manner of administration, effect of combination of medicaments, etc. A moderate dosage of each medicament (active ingredient) is, for example, about 0.005 - 2 mg/kg of body weight/day for treating dysmnesia and other cognitive impairments associated with dementia, which may be administered in one time or several times a day.

The medicine of the invention comprising a combination of an acetylcholinesterase inhibitor and a 5-substituted-3-oxadiazolyl-1,6-naphthyridin-2(1H)-one derivative of the formula (I) or a physiologically acceptable acid addition salt thereof can be used for treating dysmnesia associated with dementia and schizophrenia, and for treating neuropsychiatric diseases causing cognitive impairment.

The dementia means a disease characterized by hypofunction of the cholinergic or the glutamatergic neuron, including neurodegenerative disorders like Alzheimer's disease and cerebrovascular disorders.

The neurodegenerative disorder includes, for example, Alzheimer's disease, Parkinson's disease, Huntington's disease, Pick's disease, progressive supranuclear palsy, corticobasal degeneration, amyotrophic lateral sclerosis, diffuse Lewy body disease, traumatic neurological disease, spinocerebellar ataxia and Down's syndrome.

The dementia caused by cerebrovascular disorder includes, for example, cerebral infarction, intracerebral hemorrhage, cerebral embolism, subarachnoid hemorrhage and chronic subdural hematoma.

### EXAMPLES

Hereinafter, methods and results of the pharmacological experiments using a combination of the invention comprising an acetylcholinesterase inhibitor and a typical compound of the formula (I) are illustrated, but are not limited thereto.

### (Pharmacological Experiments)

The pharmacological experiments were carried out with regard to ameliorating effect on spatial memory disorder (dysmnesia) induced by scopolamine (a competitive antagonist for acetylcholine receptor) and by MK-801 (a noncompetitive antagonist for NMDA receptor, which is a subtype of glutamic acid receptor). These pharmacological experiments are useful as a test method for evaluating therapeutic effects in neuropsychiatric diseases causing dysmnesia and other cognitive impairments.

The reagents, the acetylcholinesterase inhibitor and the representative test compounds of the formula (I) used in the pharmacological experiments were the following compounds.

Scopolamine hydrobromide (hereinafter, abbreviated to just "scopolamine") is described in, for example, Merck Index, 13th Edition, 8481 (2001), and is also commercially available, (for example, Sigma Aldrich Japan).

MK-801 (dizocilipine maleate) is described in, for example, Merck Index, 13th Edition, 3422 (2001), and is also commercially available, (for example, Sigma Aldrich Japan).

Donepezil hydrochloride is described in, for example, Merk Index, 13th Edition, 3453 (2001) as E-2020, and is also commercially available, (Aricept^{®} Tablet, Eisai).

Test compound A: 3-(5-methyl-1,2,4-oxadiazol-3-yl)-5-(3-methoxyphenyl)-1,6-naphthyridin-2(1H)-one (the compound in Example 86 of WO 99/003857).

Test compound B: 3-(3-cyclopropyl-1,2,4-oxadiazol-5-yl)-5-(3-thienyl)-1,6-naphthyridin-2(1H)-one (the compound in Example 247 of WO 99/003857).

### (Test method)

The pharmacological experiment to investigate ameliorating effect on spatial memory disorder induced by scopolamine was carried out by systemic administration to animals according to the following method of Itoh, T. et al. In addition, the pharmacological experiment to investigate ameliorating effect on spatial memory disorder induced by MK-801 was carried out by systemic administration to animals according to the following method of Maurice, T. et al.

The spatial memory test using Y-maze apparatus which was selected for the pharmacological experiment is a test to utilize the behavioral property of animals to enter into a new arm, avoiding the arm that they entered into just before (alternation behavior). This method is often used in order to study spatial working memory.

### Experiment 1: Ameliorating effect on spatial memory disorder induced by scopolamine.

This experiment was carried out according to the method of Itoh, J., et al. [Eur. J. Pharmacol., 236, pp. 341-345 (1993)].

Ten to twelve ddY male mice weighing 28 - 34 g were used per one group in the experiment. A solution of scopolamine in physiological saline (concentration: 0.06 mg/ml) was subcutaneously administered to mice in a volume of 0.1 ml/10 g of body weight, i.e., 0.6 mg/kg. One hour before scopolamine administration, the groups of mice to which each test compound should be singly administered were orally treated with test compound A, test compound B or donepezil suspended in 0.5 % tragacanth solution in a volume of 0.1 ml/10 g of body weight; the group of mice to which a combination comprising each test compound and donepezil should be administered was orally treated with a mixture solution of test compound A or B, and donepezil in a volume of 1 mg/kg [this dosage is one tenth of the minimum effective dose (MED) at which the significant ameliorating effect of the test compounds on scopolamine-induced spatial memory disorder is observed at 5% significance level in the rate of alternation behavior, and therefore does not affect a significant effect to the alternation behavior] suspended in 0.5 % tragacanth solution in a volume of 0.1 ml/10 g of body weight; and the animals in the amnesia control group and the vehicle control group orally received a 0.5 % tragacanth solution in a volume of 0.1 ml/10 g of body weight. However, the vehicle control group was injected with physiological saline instead of scopolamine. Thirty minutes after the administration of scopolamine, the mice were placed at the end of the arm A of the Y-maze apparatus which was composed of three black acrylic trapezoid arms (bottom width : 3 cm, height of side wall : 12 cm, width of opened ceiling : 10 cm, length : 40 cm), wherein said three arms were connected at the one end of each arm to form Y-shape and another ends were closed, and the three arms were differentiated by the names as A, B and C respectively, and allowed to search freely the maze for 8 minutes. When the mouse was checked to enter into an arm at the length of not less than 10 cm from the entrance of the arm, the name of the arm (A, B or C) was recorded. For the final data gotten, the ratio of the number of the alternation behavior to that of the entries (obtained by subtracting 2 from the total number of arm entries) was estimated as the rate of alternation behavior.

In statistical analysis, the rate of alternation behavior in the amnesia control group was compared with that in the vehicle control group by the Wilcoxon rank sum test and it was checked if a significant amnesia was induced in the amnesia control group. Next, the efficacy of the test compounds in single use or in combination use of the test compounds with donepezil was evaluated in comparison between the amnesia control group and the single-administration groups using each one of the test compound A, the test compound B or donepezil; or in comparison between the amnesia control group and the combination groups of the test compound A or B and donepezil, by the nonparametric Dunnett multiple comparison test. The statistical calculation was carried out with SAS^{®} System (Release 8.02, SAS Institute Inc.) and Preclinical Package Version 5.0 (SAS Institute Japan Ltd.). Table 1 shows the minimum effective dose (MED) of the test compounds to ameliorate scopolamine-induced spatial memory disorder.

The rate of alternation behavior in the amnesia control group treated with scopolamine significantly decreased to 39 - 46 %, while 63 - 70 % in the vehicle control group, and hence, it was confirmed that the hypofunction of cholinergic system caused spatial memory disorder.

**Table 1. Ameliorating effect on spatial memory disorder induced by scopolamine**

| Test compound | Single administration [MED (mg/kg)] | # [MED (mg/kg)] |
|---|---|---|
| A | 0.3 | 0.03 |
| B | 0.1 | 0.01 |
| Donepezil | 10 | - |

| | | |
|---|---|---|
| #: Combined administration with donepezil in a volume of 1 mg/kg | | |

In the experiment of single administration of the test compound(s), the spatial memory disorder induced by scopolamine was significantly ameliorated when 0.3 mg/kg or more of test compound A was administered or when 0.1 mg/kg or more of test compound B was administered. On the other hand, the spatial memory disorder induced by scopolamine was significantly ameliorated when 10 mg/kg or more of donepezil was administered.

Through the concomitant (combined) administration, i.e., in the combination with 1 mg/kg of donepezil, which is the dosage at which the spatial memory disorder induced by scopolamine was not ameliorated by single administration thereof, the minimum effective dose (MED) of test compound A was shifted from 0.3 mg/kg in the single administration to 0.03 mg/kg in the concomitant administration; and that of test compound B was shifted from 0.1 mg/kg in the single administration to 0.01 mg/kg in the concomitant administration. The potency of the test compounds was enhanced ten times through the concomitant administration. That is to say, the concomitant administration brought a synergistic effect on ameliorating spatial memory disorder induced by scopolamine.

### Experiment 2: Ameliorating effect on spatial memory disorder induced by MK-801.

This experiment was carried out according to the method of Maurice, T., et al. [Brain Res., 647, pp.44-56 (1994)].

Ten to twelve ddY male mice weighing 26 - 36 g were used per one group in the experiment. A solution of MK-801 in physiological saline (concentration: 0.01 mg/ml) was subcutaneously administered to mice in a volume of 0.1 ml/10 g of body weight, i.e., 0.1 mg/kg. One hour before MK-801 administration, the groups of mice to which each test compound should be singly administered were orally treated with test compound A, test compound B or donepezil suspended in 0.5 % tragacanth solution in a volume of 0.1 ml/10 g of body weight; and the animals in the amnesia control group and the vehicle control group orally received a 0.5 % tragacanth solution in a volume of 0.1 ml/10 g of body weight. However, the vehicle control group was injected with physiological saline instead of MK-801. Twenty minutes after administration of MK-801, the mice were placed at the end of the arm A of the Y-maze apparatus which was composed of three black acrylic trapezoid arms (bottom width : 3 cm, height of side wall : 12 cm, width of opened ceiling : 10 cm, length : 40 cm), wherein said three arms were connected at the one end of each arm to form Y-shape and another ends were closed, and the three arms were differentiated by the names as A, B and C respectively, and allowed to search freely the maze for 8 minutes. When the mouse was checked to enter into an arm at the length of not less than 10 cm from the entrance of the arm, the name of the arm (A, B or C) was recorded. For the final data gotten, the ratio of the number of the alternation behavior to that of the entries (obtained by subtracting 2 from the total number of arm entries) was estimated as the rate of alternation behavior.

In statistical analysis, the rate of alternation behavior in the amnesia control group was compared with that in the vehicle control group by the Wilcoxon rank sum test and it was checked if a significant amnesia was induced in the amnesia control group. Next, the efficacy of the test compounds was evaluated in comparison between the amnesia control group and each group of test compound A, test compound B or donepezil by the nonparametric Dunnett multiple comparison test. The statistical calculation was carried out with SAS^{®} System (Release 8.02, SAS Institute Inc.) and Preclinical Package Version 5.0 (SAS Institute Japan Ltd.). Table 2 shows the minimum effective dose (MED) of the test compounds to significantly ameliorate MK-801-induced spatial memory disorder.

The rate of alternation behavior in the amnesia control group with MK-801 significantly decreased to 45 - 47 %, while 65 - 73 % in the vehicle control group, and hence, it was confirmed that the hypofunction of glutamatergic system caused spatial memory disorder.

**Table 2. Ameliorating effect on spatial memory disorder induced by MK-801**

| Test compound | [MED (mg/kg)] |
|---|---|
| A | 0.3 |
| B | 0.1 |
| Donepezil | >15 |

The spatial memory disorder induced by MK-801 was significantly ameliorated when 0.3 mg/kg or more of test compound A was administered or when 0.1 mg/kg or more of test compound B was administered. However, the spatial memory disorder induced by MK-801 was not ameliorated by donepezil.

As it is obvious from Experiments 1 and 2, the 5-substituted-3-oxadiazolyl-1,6-naphthyridin-2(1H)-one derivatives of the formula (I) (representative test compounds A and B) exhibited markedly potent synergistic ameliorating effect on dysmnesia (memory disorder) induced by systemic administration of scopolamine to animals through the concomitant administration with an acetylcholinesterase inhibitor. Furthermore, the single administration of the above-mentioned compounds brought markedly potent ameliorating effect on dysmnesia (memory disorder) induced by systemic administration of MK-801, which could not be ameliorated by acetylcholinesterase inhibitor (e.g. donepezil).

### INDUSTRIAL APPLICABILITY

The medicine of the present invention comprising a combination of an acetylcholinesterase inhibitor and a 5-substituted-3-oxadiazolyl-1,6-naphthyridin-2(1H)-one derivative of the formula (I) or a physiologically acceptable acid addition salt thereof is useful for treating neuropsychiatric diseases especially dementia causing dysmnesia and other cognitive impairments characterized by hypofunction of the cholinergic or the glutamatergic neuron.

## Claims

1. A medicine comprising a combination of an acetylcholinesterase inhibitor and
at least one compound selected from 5-substituted-3-oxadiazolyl-1,6-naphthyridin-2(1H)-one derivatives of the formula (I): 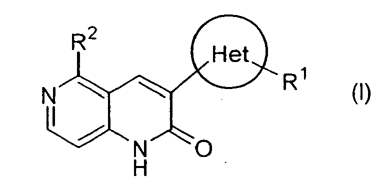 wherein Het is an oxadiazolyl group;
R¹ is hydrogen atom, a lower alkyl group, a lower cycloalkyl group, trifluoromethyl group, a lower alkenyl group, a lower alkynyl group, a lower alkoxy group, a lower alkoxy-lower alkyl group, a hydroxy-lower alkyl group, a substituted or unsubstituted aryl group, or a substituted or unsubstituted heteroaryl group; and
R² is hydrogen atom, a lower alkyl group, a lower cycloalkyl group, a lower cycloalkylmethyl group, a lower alkenyl group, a lower cycloalkenyl group, a lower alkynyl group, a substituted or unsubstituted aryl group, or a substituted or unsubstituted heteroaryl group, and physiologically acceptable acid addition salts thereof.

2. The medicine according to claim 1 wherein R¹ of the formula (I) is a C₁-C₃ alkyl group, a C₃-C₄ cycloalkyl group, or a C₂-C₃ alkenyl group; and
R² of the formula (I) is hydrogen atom, a C₁-C₄ alkyl group, a C₃-C₆ cycloalkyl group, a substituted or unsubstituted aryl group, or a substituted or unsubstituted heteroaryl group.

3. The medicine according to claim 2 wherein R¹ of the formula (I) is a C₁-C₃ alkyl group or a C₃-C₄ cycloalkyl group; and
R² of the formula (I) is hydrogen atom, a C₁-C₃ alkyl group, a C₃-C₄ cycloalkyl group, a substituted or unsubstituted phenyl group, or a substituted or unsubstituted heteroaryl group.

4. A medicine comprising a combination of an acetylcholinesterase inhibitor and
at least one 5-substituted-3-oxadiazolyl-1,6-naphthyridin-2(1H)-one derivative selected from:
3-(5-ethyl-1,2,4-oxadiazol-3-yl)-5-(2-methylcyclopropyl)-1,6-naphthyridin-2(1H)-one,
3-(5-methyl-1,2,4-oxadiazol-3-yl)-5-(2-methylphenyl)-1,6-naphthyridin-2(1H)-one,
3-(5-methyl-1,2,4-oxadiazol-3-yl)-5-(4-methoxyphenyl)-1,6-naphthyridin-2(1H)-one,
3-(5-ethyl-1,2,4-oxadiazol-3-yl)-5-(2-thienyl)-1,6-naphthyridin-2(1H)-one,
3-(5-methyl-1,2,4-oxadiazol-3-yl)-5-(4-pyridyl)-1,6-naphthyridin-2(1H)-one,
3-(3-ethyl-1,2,4-oxadiazol-5-yl)-5-methyl-1,6-naphthyridin-2(1H)-one,
3-(3-ethyl-1,2,4-oxadiazol-5-yl)-5-(3-fluorophenyl)-1,6-naphthyridin-2(1H)-one,
3-(3-methyl-1,2,4-oxadiazol-5-yl)-5-(3-methylphenyl)-1,6-naphthyridin-2(1H)-one,
3-(3-methyl-1,2,4-oxadiazol-5-yl)-5-(3-methoxyphenyl)-1,6-naphthyridin-2(1H)-one,
3-(3-ethyl-1,2,4-oxadiazol-5-yl)-5-(4-methoxyphenyl)-1,6-naphthyridin-2(1H)-one, and
3-(3-ethyl-1,2,4-oxadiazol-5-yl)-5-(4-pyridyl)-1,6-naphthyridin-2(1H)-one,
or a physiologically acceptable acid addition salt thereof.

5. A medicine comprising a combination of an acetylcholinesterase inhibitor and
3-(5-methyl-1,2,4-oxadiazol-3-yl)-5-(3-methoxyphenyl)-1,6-naphthyridin-2(1H)-one or a physiologically acceptable acid addition salt thereof.

6. A medicine comprising a combination of an acetylcholinesterase inhibitor and
3-(3-cyclopropyl-1,2,4-oxadiazol-5-yl)-5-(3-thienyl)-1,6-naphthyridin-2(1H)-one or a physiologically acceptable acid addition salt thereof.

7. The medicine according to any one of claims 1 to 6 wherein the acetylcholinesterase inhibitor is at least one compound selected from the group consisting of donepezil hydrochloride, tacrine hydrochloride, rivastigmine tartrate and galantamine hydrobromide.

8. The medicine according to any one of claims 1 to 6 wherein the acetylcholinesterase inhibitor is donepezil hydrochloride.

9. A method for treating dysmnesia and other cognitive impairments associated with neuropsychiatric diseases, comprising administering a therapeutically effective amount of the medicine as set forth in any one of claims 1 to 8 to a patient suffering therefrom.

10. The method according to claim 9 wherein the neuropsychiatric disease is dementia or schizophrenia.

11. The method according to claim 10 wherein the dementia is a disease **characterized by** hypofunction of the cholinergic or the glutamatergic neuron.

12. The method according to claim 10 or 11 wherein the dementia is a disease caused by neurodegenerative disorder or cerebrovascular disorder.

13. The method according to claim 12 wherein the neurodegenerative disorder is Alzheimer's disease, Parkinson's disease, Huntington's disease, Pick's disease, progressive supranuclear palsy, corticobasal degeneration, amyotrophic lateral sclerosis, diffuse Lewy body disease, traumatic neurological disease, spinocerebellar ataxia, or Down's syndrome.

14. The method according to claim 12 wherein the disease caused by cerebrovascular disorder is cerebral infarction, intracerebral hemorrhage, cerebral embolism, subarachnoid hemorrhage or chronic subdural hematoma.

15. A method for treating Alzheimer's disease comprising administering a therapeutically effective amount of the medicine as set forth in any one of claims 1 to 8 to a patient in need thereof.

16. The method according to any one of claims 9 to 15 wherein the acetylcholinesterase inhibitor and the 5-substituted-3-oxadiazolyl-1,6-naphthyridin-2(1H)-one derivative or a physiologically acceptable acid addition salt thereof are administered simultaneously or sequentially.

17. Use of a combination of an acetylcholinesterase inhibitor and a 5-substituted-3-oxadiazolyl-1,6-naphthyridin-2(1H)-one derivative or a physiologically acceptable acid addition salt thereof as set forth in any one of claims 1 to 6, for the manufacture of a medicine for treating dysmnesia and other cognitive impairments associated with neuropsychiatric diseases.

18. Use of a combination of an acetylcholinesterase inhibitor and a 5-substituted-3-oxadiazolyl-1, 6-naphthyridin-2(1H)-one derivative or a physiologically acceptable acid addition salt thereof as set forth in any one of claims 1 to 6, for the manufacture of a medicament for treating dementia.

19. The use according to claim 18 wherein the dementia is a disease caused by Alzheimer's disease or cerebrovascular disorder.
